# EUROPÄISCHE PATENTSCHRIFT

(11) **EP 0 753 300 B1**
(45) Veröffentlichungstag und Bekanntmachung des Hinweises auf die Patenterteilung: **22.09.1999**
(21) Anmeldenummer: 96110339.7
(22) Anmeldetag: 27.06.1996
(51) Int. Cl.: A61K 31/565

(54) **Verwendung von Steroiden mit antioxidativen Eigenschaften zur Herstellung von pharmazeutischen Präparaten zur medikamentösen Substitution beim Mann**
Use of antioxidative steroids for the manufacture of pharmaceutical preparations for substition therapy in man
Utilisation de stéroides antioxidants pour la préparation de médicaments pour le traitement de substition médicamenteuse chez l'homme

(30) Priorität: 08.07.1995 DE 19524937
(43) Veröffentlichungstag der Anmeldung: 15.01.1997
(73) Patentinhaber: Jenapharm GmbH & Co. KG, 07745 Jena (DE)
(72) Erfinder: Oettel, Michael, Prof. Dr., 07743 Jena (DE); Römer, Wolfgang, Prof. Dr., 07749 Jena (DE); Hübler, Doris, Dr., 07407 Schmieden (DE); Schröder, Jens, 07747 Jena (DE); Schwarz, Sigfrid, Prof. Dr., 07743 Jena (DE); Droescher, Peter, Dr., 99423 Weimar (DE)
(74) Vertreter: Cramer, Eva-Maria

(56) Entgegenhaltungen:
- DE-C- 4 338 314
- US-A- 3 626 061
- FEBS LETT., Bd. 332, Nr. 1-2, Oktober 1993, Seiten 159-163, XP000605413 WISEMAN, H. ET AL.: "carcinogenic antioxidants"
- LIPIDS, Bd. 30, Nr. 2, Februar 1995, Seiten 141-146, XP000604488 LACORT, M. ET AL.: "protective effect of estrogens and catecholestrogens against peroxidative membrane damage in-vitro"
- ARTERIOSCLEROSIS, THROMBOSIS AND VASCULAR BIOLOGY, Bd. 15, Nr. 7, Juli 1995, Seiten 837-846, XP000605741 SULISTIYANI, S.J. ET AL.: "effect of 17alpha-dihydroequilin sulfate, a conjugated equine estrogen, and ethynylestradiol on atherosclerosis in cholesterol-fed rabbits"
- ATHEROSCLEROSIS, Bd. 109, Nr. 1-2, Oktober 1994, Seiten 102-103, XP000605805 SULISTIYANI ET AL.: "effect of 17alpha-dihydroequilin sulfate, a water soluble estrogen of premarin, on atherosclerosis in cholesterol-fed rabbits"

## Beschreibung

Die Erfindung betrifft die Verwendung von Steroiden mit antioxidativen Eigenschaften - 17α-Estradiol, dessen chemisch modifizierte Derivate oder Ester des 17α-Estradiol oder deren chemisch modifizierte Derivate - ausgenommen Ethinylestradiol- zur Herstellung pharmazeutischer Präparate zur Therapie chronisch-degenerativer Erkrankungen im Gehirn von Atherosklerose und Arthritis/Arthrose beim Mann. hormonellen Substitution beim Mann.

Aus der Fach- und Patentliteratur ist bekannt, daß reaktive Sauerstoffspezies (ROS), freie Sauerstoffradikale und weitere Radikalformen eine wichtige Rolle bei der Entstehung vielfältiger Zellschädigungen spielen. Dabei ist u.a. die Lipidperoxidation, die Oxidation von Low Density Lipoprotein (LDL)-Cholesterol und die Xanthinoxidase in Verbindung mit irreversiblen Membran- und Endothelschädigungen Ausgangspunkt radikalvermittelter Zellschädigungen.

Die Patentschriften WO 87/01706 A2; WO 91/11453 A2;
EP 0389 368 A1; EP 0389 369 B1; EP 0389 370 A1 und
FR 2 640 977 beschreiben beispielsweise Steroide mit "radikalfangenden" Eigenschaften (antioxidative Eigenschaften).

In J. Phys. Org. Chem. 3 (1990), 309-315 wird dargestellt, daß Estrogene, speziell Catecholestrogene, als Radikalfänger agieren können. Estradiol, Estron, Estriol und 2-Hydroxy-estradiol hemmen die Peroxidation in vitro und in vivo.

Weiterhin beschreibt die Patentschrift DE 43 38 314 C1 pharmazeutische Präparate zur Prophylaxe und Therapie radikalvermittelter Zellschädigungen, welche aus Steroiden mit phenolischer A-Ring-Struktur bestehen.
Aus dieser Schrift ist ersichtlich, daß das Maß der radikalfangenden (antioxidativen) Wirksamkeit unabhängig ist vom Maß der estrogenen Wirksamkeit der jeweiligen Verbindungen. Beispielsweise bewegt sich die In-vitro-Hemmwirkung auf die Lipidperoxidation für mehrere Verbindungen im gleichen Maße, aber die estrogene Wirksamkeit ist unterschiedlich.
Jedoch ist zu bemerken, daß antioxidative Eigenschaften dort zu verzeichnen sind, wo ein auch bestimmtes Maß an Estrogenität der jeweiligen Verbindung vorhanden ist.

Weiterhin dokumentiert Mooradian A.D in: Antioxidant properties of steroids, J. Steroid Biochem. Mol. Bio. 45:5O9-511 (1993)] die anti-oxidativen Eigenschaften von 17β-Estradiol, seinen verschiedenen Derivaten und Estern, wie z. B. Ethinylestradiol und die Katecholestrogene. Diese Eigenschaften tragen wesentlich dazu bei, daß die sogenannte Hormonsubstitution in der Postmenopause bei Frauen sehr deutlich das Herz-Kreislauf-Risiko senkt.

Abgesehen vom Hypogonadismus (zu geringe Androgenproduktion in den Hoden; nur etwa 7 % aller Männer) ist es bisher nicht gelungen, beim alternden Mann ein ähnliches Hormonsubstitutionsprinzip wie für die postmenopausale Frau zu entwickeln. Psychische und physische Verschlechterungen, die beim Mann um die 5O plötzlich auftreten, wurden bisher als sogenanntes Klimakterium virile bezeichnet, obwohl an den Hodenparametern und hinsichtlich der Gonadotropinsekretion keine Auffälligkeiten nachzuweisen waren [Umbreit K: Was tun bei "Klimakterium virile"? TW Gynäkologie 8:22O-229 (1995)].

Emons G, Knuppen R, Ball P legen in: 4-Hydroxyestradiol-17β and 4-hydroxyestradiol-17α: Comparative studies on central and peripheral effects of two epimeric catecholestrogens, Endocrinology 109:1799 (1979); die im Vergleich zum 17β-Estradiol um mehrere Größenordnungen geringere Affinität des 17α-Estradiol zum zytoplasmatischen bzw. nuklearen Estrogenrezeptor dar.

Auch Merriam G, Mac Lusky N., Picard M., Naftolin F beschreiben diesen Sachverhalt in: Comparative properties of the catechol estrogens I: Methylation by catechol-O-methyltransferase and binding to cytosol estrogen receptors. Steroids 36:1-11 (198O).

Es kann daher eine fehlende estrogene Wirksamkeit von 17α-Estradiol erklärt werden.

Es ist weiterhin bekannt, daß es eine positive Korrelation von freien Radikalen und epileptogenen, krampfauslösenden Prozessen im Gehirn gibt.
Levy SL, Burnham WM, Bishai A, Hwang PA beschreiben in: The anticonvulsant effects of vitamin E: a further evalution. Can. J. Neurol. Sci. 19:201-203 (1992); Levy SL, Burnham WM, Bishai A, Hwang PA: An evalution of the anticonvulsant effects of vitamin E. Epilepsy Res. 6:12-17 (1990); Santiago LA, Osato JA, Hiramatsu M, Edamatsu R, Mori A: Free radical scavanging action of Bio-catalyzer alpha.rho No. 11 (Bionormalyzer) and its by-product. Free Radic. Biol. Med. *11*:379-383 (1991), daß Radikalscavenger antikonvulsiv wirken und z.B. bei Mäusen, bei denen durch mehrmalige Gabe von Pentetrazol ein Kindling-Phänomen mit einem typischen Krampfverhalten ausgebildet wird, dieses Krampfmuster beeinflussen.
Nachteilig dabei ist, daß nur durch die Applikation von hohen Dosen und/oder eine lange Substanzapplikation eine Beeinflussung des Krampfmusters erfolgen kann.

Die Patentschrift DE 43 38 314 C1 beschreibt pharmazeutische Präparate, Steroide mit phenolischer A-Ring-Struktur enthaltend. Da die aufgezeigten Verbindungen nachweislich sowohl Inhibitoren der Lipidperoxidation als auch Inhibitoren der LDL-Oxidation darstellen, sind sie geeignet zur Prophylaxe und Therapie radikalvermittelter Zellschädigungen.
Ein Nachweis der antioxidativen biologischen Wirksamkeit im männlichen Organismus ist nicht aufgezeigt.

Febs.Lett., 332(1-2), Oktober 1993, Seiten 159-163, Wiseman H. et al., beschreibt die antioxidative Wirkung von Ethinylestradiol (=17α-Ethinylestradiol) und deren Wirksamkeit bei der Hemmung der Lipidperoxidation.
Auch Lipids, 30(2), 1995, Seiten 141-146, Lacort M., et al., beschreibt die antioxidativen Effekte von Ethinylestradiol, jedoch vor spontan auftretender Peroxidation.
In beiden Schriften ist kein Nachweis der biologischen Wirksamkeit im männlichen Organismus aufgezeigt und es ist eine Ethinylestradiol-Therapie im männlichen Organismus nicht geeignet.

US-A-3 626 061 offenbart 17α-alkylierte Estradiolverbindungen und deren Nutzen bei der Reduzierung des Cholesterolspiegels im Blut; ein Nachweis der biologischen Wirksamkeit im männlichen Organismus ist nicht aufgezeigt.

Arteriosclerosis, Thrombosis und Vascular Biology, 15(7), Juli 1995, Seiten 837-846, Sulistiyani S.J.A. et al., und Atherosclerosis, 109(1-2), Oktober 1994, Seiten 102-103, Sulistiyani S.J.A. et al., beziehen sich auf den protektiven Effekt von 17α-Dihydroequilinsulfat (DHES) und Ethinylestradiol bei der Entwicklung von Atherosklerose.
Der Effekt wird allerdings nur für weibliche Kaninchen belegt.

Nachweislich ist aus der Fach- und Patentliteratur eine Anwendung von 17α-Estradiol und gezielt synthetisierter Derivate des 17α-Estradiols zur Hormonsubstitution beim alternden Mann, welche verbunden ist mit der präventiven und therapeutischen Beeinflussung derartiger Leistungsminderungen und Krankheitsbilder, bei deren Entstehung ursächlich überproportional freie Radikale diskutiert werden, nicht erkennbar und schlußfolgernd.

Der Erfindung liegt die Aufgabe zugrunde, eine für den Mann hormonelle Substitutionstherapie mittels Estrogene aufzuzeigen und die genannten Nachteile zu vermeiden.

Die Aufgabe wird erfindungsgemäß durch die Verwendung von mindestens einem 17α-Estradiol, dessen chemisch modifizierten Derivaten oder den Estern des 17α-Estradiol oder deren chemisch modifizierten Derivaten ausgenommen Ethinylestradiol, zur Herstellung von pharmazeutischen Präparaten zur medikamentösen hormonellen Substitution beim Mann gelöst.

Es wurde festgestellt, daß überraschenderweise für den Mann die Möglichkeit einer Substitutionstherapie mittels Estrogene gefunden wurde ohne die für den Mann unerwünschten Nebenwirkungen (Genomvermittelte) einer klassischen Hormontherapie.

Vorteilhafte Ausgestaltungen der Erfindung sind die Verbindungen 17α -Estradiol, 8-Dehydro-17α-estradiol (J 811) und 14,15α-Methylen-8-dehydro-17α-estradiol (J 861).

Gegenstand der vorliegenden Erfindung sind auch pharmazeutische Präparate zur oralen und parenteralen, incl. topischen, rektalen, subcutanen, intravenösen, intramuskulären, intraperitonealen, intranasalen, intravaginalen, intrabukkalen oder sublingualen Applikation, die neben üblichen Träger- und Verdünnungsmitteln eine in dem Anspruch 1 aufgezeigte Verbindung als Wirkstoff enthalten.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die Vorteile der Erfindung ergeben sich im wesentlichen durch die Verwendung von Steroiden mit antioxidativen Eigenschaften zur Herstellung von Arzneimitteln zur medikamentösen hormonellen Substitution beim Mann,
- mit Wirkstoffen, deren Wirkprofil den präventiven und therapeutischen Einsatz bei Männern mit einem erhöhten Risiko für Erkrankungen, bei denen freie Sauerstoffradikale für Entstehung und/oder Unterhalt verantwortlich zu machen sind -beispielsweise Atherosklerose, Arthritis/Athrose und chronisch-degenerative Prozesse im Gehirn -, ermöglicht
- und eine Substitutionstherapie mittels Estrogene gewährleistet ohne die für den Mann unerwünschten Nebenwirkungen (Genomvermittelte) einer klassischen Hormontherapie.
Die vorteilhaften Wirkungen der erfindungsgemäßen Systeme werden anhand der Ergebnisse von endokrinpharmakologischen In-vitro- und In-vivo- Untersuchungen aufgezeigt.
Ergebnisse einer klinischen Studie belegen ebenfalls die vorteilhafte Wirkung der erfindungsgemäßen Systeme.

### Hemmung der Expression ausgewählter Adhäsionsmoleküle

Die Hemmung der TNFα-induzierten VCAM-1 und ICAM-1-Expression in kultivierten Endothelzellen humanen Ursprungs wurde nach Jilma B, Eichler H-G, Breiteneder H, Wolzt M, Aringer M, Graninger W, Röhrer C, Veitl M, Wagner OF: Effects of 17β-estradiol on circulating adhesion molecules. J. Clin. Endocrinol. Metab. *7*:1619-1624 (1994) ausgeführt.

Als Vergleich zu den erfindungsgemäßen Systemen diente 17β-Estradiol, welches mit (x) gekennzeichnet wurde.

**Tabelle 1**

| Die Hemmung der TNFα-induzierten VCAM-1- und ICAM-1-Expression in kultivierten Endothelzellen humanen Ursprungs durch Estrogene. | | |
|---|---|---|
| Prüfsubstanzen | Hemmung¹^{,}² der Expression von | |
| | VCAM-1 | ICAM-1 |
| 17α-Estradiol | + + | + + |
| J 861 - 14,15α-Methylen-8-dehydro-17α-estradiol | + + + | + + + |
| 17β-Estradiol (x) | + + | + |

| | | |
|---|---|---|
| 1) Bewertungsparameter ist eine histologische Beurteilung | | |
| 2) Bewertungsskala: +: wenig wirksam; ++: gut wirksam; +++: sehr gut wirksam | | |

Wie in Tabelle 1 veranschaulicht, verhält sich auch in diesem Modell - hinsichtlich die Hemmung der TNFα-induzierten VCAM-1 und ICAM-1-Expression - 17α-Estradiol wie 17β-Estradiol und die Substanz J 861 - 14,15α-Methylen-8-dehydro-17α-estradiol - zeigt die stärksten Hemmeffekte.

### In-vivo-Befunde

Die Verbindungen wurden in einer Reihe von In- vivo-Testmodellen zur Erfassung antioxidativer Wirkungen getestet.

Die positive Korrelation von freien Radikalen und epileptogenen, krampfauslösenden Prozessen im Gehirn werden nach Levy SL et al:, The anticonvulsant effects of vitamin E: a further evalution. Can. J. Neurol. Sci. 19:201-203 (1992); und: An evalution of the anticonvulsant effects of vitamin E. Epilepsy Res. 6:12-17 (1990); Santiago LA et al: Free radical scavenging action of Bio-catalyzer alpha.rho No. 11 (Bionormalyzer) and its by-product : Free Rad. Bio. Med. 11:379-383 (1991), ausgeführt.

**Tabelle 2**

| Wirkung von J 861 auf die einzelnen Krampfstadien des Pentetrazol-Kindlings an männlichen Mäusen im Vergleich zu dem Referenz-Antiepileptikum Carbamazepin. | | | | | | | |
|---|---|---|---|---|---|---|---|
| Substanz | Dosis [mg/kg] | Krampfstadien¹ | | | | | |
| | | 1/2 | | 3 | | 4/5/6 | |
| | | LZ_{[s]} | Kl | LZ_{[s]} | Kl | LZ_{[s]} | Kl |
| J 861 | 1,0 | - | - | - | +++ | + | + |
| | 10 | - | - | - | ++ | ++ | + |
| | 100 | - | - | - | ++ | ++ | + |
| Carbamazepin 24 | | + | - | + | - | ++ | + |

| | | | | | | | |
|---|---|---|---|---|---|---|---|
| 1) Bewertung der Wirkung: - kein Effekt + Wirkung nachgewiesen ++ gute Wirkung +++ sehr gute Wirkung LZ_{[s]} Latenzzeit bis zur Ausprägung der Krämpfe erhöht Kl Krampfindex gesenkt | | | | | | | |

Aus Tabelle 2 ist ersichtlich, daß J 861 - 14,15α-Methylen-8-dehydro-17α-estradiol z.B. bereits in einer Dosierung von 1 mg/kg nach einmaliger oraler Gabe eine Stunde vor der krampfauslösenden Pentetrazol-Injektion an männlichen Mäusen das Krampfstadium 3 hemmt. In steigender Dosierung und bei längerer Substanzapplikation werden auch die anderen Krampfstadien beeinflußt.

Dieses Beispiel zeigt, daß die erfindungsgemäßen Substanzen nach oraler Applikation in niedrigen Dosen die Blut-Hirn-Schranke in kurzer Zeit überwinden und durch freie Radikale modulierte degenerative Prozesse im Gehirn positiv beeinflussen. Ausgehend von den In-vitro-Ergebnissen hinsichtlich des Verhaltens der erfindungsgemäß beschriebenen Estrogene am Kainat-Rezeptor von Gliazellen, ist dies ein weiterer Hinweis, daß nichtgenomische Estrogenwirkungen über den GABAergen Rezeptorkomplex vermittelt werden können.

Die Adjuvansarthritis bei Lewis-Ratten ist ein gut bekanntes und etabliertes Modell für die chronische Rheumatoid-Arthritis des Menschen [Newbould BB: Chemotherapy of arthritis induced in rats by mycobacterial adjuvant. Br. J. Pharmacol. 21:127-136 (1963); Hirschelmann R: Die 6-Sulfanilamidoindazol-Arthritis und die Adjuvansarthritis der Ratte als Modelle zur Testung von Antiphlogistika. Dissertation B (Habil.-Schrift), Halle, 1976]

Die erfindungsgemäßen Substanzen wurden im Vergleich zu 17β-Estradiol in diesem Modell an männlichen Ratten (8 Tiere pro Gruppe) über 21 Tage nach oraler Applikation getestet. Die Arthritis wurde durch Injektion von 0,1 ml Freund'sches Adjuvans in die linke Hinterpfote ausgelöst. Prüfparameter waren die Hemmung der Schwellung der linken Hinterpfote und der Grad der Sekundärarthritis durch Beurteilung der Schwellung der drei anderen Pfoten.

**Tabelle 3**

| Wirkung der Substanzen im Vergleich zu 17β-Estradiol (x) in der Adjuvansarthritis der männlichen Ratte. | | | |
|---|---|---|---|
| Substanz | Dosierung [mg/kg] | % Hemmung Primärarthritis max. Wirkung | Sekundärarthritis Score ¹ |
| 17β-Estradiol (x) | 1 | 21 | 47 |
| | 10 | 35 | 36 |
| 17α-Estradiol | 1 | 36 | 43 |
| | 10 | 39 | 35 |
| J 861 | 1 | 16 | 72 |
| | 10 | 38 | 46 |
| | 25 | 61 | 38 |
| | 50 | 47 | 35 |

| | | | |
|---|---|---|---|
| 1) Score: Beurteilung der Sekundärarthritis an den drei nichtinjizierten Pfoten 0 = keine, 1 = leichte, 2 = mittelstarke, 3 = starke Entzündung. Maximalpunktzahl je Tier 3 x 3 = 9, das sind bei 8 Tieren max. 72 je Gruppe (arthritische Kontrolle). | | | |

Aus Tabelle 3 wird ersichtlich, daß 17α-Estradiol und J 861 in etwa die gleiche antiarthritische Wirkung wie 17β-Estradiol besitzen. Besonders auffällig ist die Wirksamkeit in späteren Stadien der Arthritis.

Auch im Carrageeninpfotenödem der männlichen Ratte tritt der maximale Hemmeffekt erst 24 Stunden nach Provokation des Ödems auf, wenn der Entzündungsprozeß nicht mehr akut ist und nicht-steroidale Antiphlogistika wie Diclofenac-Natrium nur noch geringe Hemmeffekte zeigen.

Während in der akuten Phase in beiden Modellen die antiinflammatorischen Effekte relativ schwach ausgeprägt blieben, war in der sogenannten chronischen Phase immer eine signifikante Entzündungshemmung nachweisbar. Dieser Befund korreliert auch mit der Beobachtung, daß die erfindungsgemäßen Substanzen die Akute-Phase-Proteine unbeeinflußt lassen.

### In-vitro-Befunde

Die Verbindungen wurden in einer Reihe von In- vitro-Testmodellen zur Erfassung der estrogenen Wirkung getestet.

### 1. Estrogen-Rezeptor-Affinität

Die Affinität zum Estrogen Rezeptor wurde in Kaninchen-Uterus-Cytosol bestimmt durch competitive Bindung von ³H-markiertem Tracer und der jeweils zu testenden Verbindungen in Konzentrationsreihen, die die IC₅₀ einschlossen. IC₅₀ gibt die Menge der zuzugebenden Substanz an, um eine 50%ige Hemmung der Lipidperoxidation zu erzielen Es wurden weibliche Kaninchen (weiße Neuseeländer) im Gewicht von 1,5 bis 2 kg in Barbituratnarkose getötet, die Uteri entnommen, gewaschen und unter Eiskühlung homogenisiert in TEDS-Puffer (20 mM Tris/HCl, pH 7,4; 1mM Ethylendiamintetraacetat; 2mMDithiothreitol; 250 mM Saccharose).
Das nach Zentrifugieren bei 16 000 g und 100 000 g gewonnene Cytosol wurde portionsweise schockgefroren und bei -30 °C aufbewahrt.
Das geeignet verdünnte Cytosol (Rezeptorkonzentration ca. 0,3 nM) wurde mit dem Tracer [³H]-Ethinylestradiol (3nM) und den zu prüfenden Competitoren bei 0 bis 4 °C für 18 Stunden inkubiert. Der freie Tracer wurde durch Adsorption an Aktivkohle/Dextran (1 %/0,1 %) und Zentrifugation abgetrennt und die gebundene ³H-Aktivität im Überstand gemessen.
Aus Messungen in Konzentrationsreihen (Competitoren im Bereich zwischen 1 und 200 nM wurden nach logit-log-Transformation der Bindungswerte die IC₅₀ für die Vergleichssubstanz 17β-Estradiol und für die zu testende Verbindung ermittelt.

Die relative Bindungsaffinität (RBA) errechnet sich als Quotient aus dem IC₅₀ der Vergleichssubstanz 17β-Estradiol (x) und der getesten Substanz.

**Tabelle 4**

| Estrogen-Rezeptor-Affinität ausgewählter Verbindungen im Vergleich zu 17β-Estradiol (x) | |
|---|---|
| Substanz | relative Bindungsaffinität zum Estrogen-Rezeptor (RBA) (%) |
| 17β-Estradiol (x) | 100 |
| 17α-Estradiol | 23 |
| J 811 | 21 |
| J 861 | 32 |

Aus Tabelle 4 wird ersichtlich, daß für 17α-Estradiol und seine Analoga die Affinität zum Estrogen-Rezeptor deutlich vermindert und mithin die estrogene Wirkung weit geringer ist im Vergleich zu 17β-Estradiol.

### 2. Estrogene Aktivität in gentechnisch veränderten humanen Brustkrebszellen

### Zellen und Zellkultur

Die stabil transfektierte humane Brustkrebszellinie MCF-7/2A enthält den Vektor pEMBL8+. Dieser Vektor enthält den ori des fl-Phagen und ein Fusionsgen, bestehend aus dem estrogen response element (ERE) des Vitellogenins A2 von *Xenopus* laevis, dem TK-Promotor aus dem Herpes Simplex Virus, dem Luciferase-Gen aus *Photinus pyralis* und dem PolyA-Signal aus SV40 (Meyer et al., 1994, J.Cancer Res. ClinOncol. 120:359-364).

Die Brustkrebszellinie MCF-7/2A wurde unter Routinebedingungen in Dulbecco's Medium (DM) ohne Phenolrot kultiviert, welches mit 5 % fetalem Kälberserum (FKS), 2 mM L-Glutamin, Penicillin (100 Einheiten/ml), Streptomycin (100 µg/ml) und Geneticin (350 µg/ml) versetzt wurde. Für die Stammkultur wurden die Zellen wöchentlich 1/10 passagiert. Alle Zellen wurden bei 37 °C in einer Atmosphäre mit nahezu 100%-iger relativer Luftfeuchtigkeit und einem Anteil an CO₂ von 5 Vol.% in der Luft kultiviert.
Eine Woche vor Versuchsbeginn wurden die Zellen in DM ohne Phenol-rot überführt, welches sich vom Stammhaltungsmedium dadurch unterscheidet, daß es kein Geneticin enthält und anstelle von FKS mit 10 % DCS versetzt wurde (mit Dextran-Aktivkohle behandeltes FKS). Innerhalb dieser Woche erfolgte ein dreimaliger Mediumwechsel.

### Bestimmung der estrogenen Aktivität

Zur Durchführung der Luciferaseinduktionen sind jeweils 1,5 Millionen Zellen in je 2 ml DM mit 10 % DCS, welches durch die Behandlung mit Dextran-gesättigter Aktivkohle von endogenen Steroiden befreit worden war, ausgesät worden.
Nach 24 Stunden erfolgte der Hormonzusatz in Ethanol (0,1 % Endvolumen).
Die Kontrollen enthielten gleiche Anteile an Vehikel.
Nach 48 Stunden Hormoneinwirkung erfolgte nach zweimaligem Waschen der Zellen mit PBS (ohne Calcium und Magnesium) der Zellaufschluß durch Zugabe von 180 µl Lysispuffer pro well (25 mM Trisphosphat, pH 7,8; 2mM DTT, 2mM 1,2-Diaminocyclohexan-N,N,N',N'-tetraessigsäure, 10 % Glycerol, 1 % Triton X-100).
Nach 15 Minuten Lyse bei Raumtemperatur wurde die Lösung mit den lysierten Zellen in einen Eppendorfbecher überführt und kurz zentrifugiert (Eppendorfzentrifuge; 2 min, 18000 U/min).
Der Luciferaseassay von Aliquoten der Überstände erfolgte entsprechend dem Protokoll von Promega in Luminoskan Type 391 in speziellen Mikrotiterplatten.

Die gemessenen relativen Lichteinheiten der Proben wurden auf ihren Proteingehalt standardisiert. Die Proteinbestimmung erfolgte nach Bradford (1976) mit Rinderserumalbumin als Standard.

Aus Abbildung 1 ist ersichtlich:
Die Genaktivierungsexperimente ergeben, daß die halbmaximale effektive Dosis zur Luciferaseinduktion für 17α-Estradiol ( in der Abbildung bezeichnet als 17a-Estradiol) und Analoga J 811 und J 861 im nanomolaren Bereich liegt, wogegen 17β-Estradiol im Bereich zwischen 10⁻¹⁰ M und 10⁻¹¹ M seinen halbmaximalen Effekt zeigt.
Im Gegensatz zu komplexen Reaktionen, wie zelluläre Wachstumstimulation und Steigerung des Uterusgewichtes, reflektiert die Luciferaseinduktion in den gentechnisch veränderten MCF-7/2a Zellen eine reine estrogene Antwort.
Die Dissoziation der genomischen Effekte von 17β-Estradiol zu 17α-Estradiol und den Analoga J 811 und J 861 um mehr als eine Zehnerpotenz wird in dem verwendeten humanen Zellsystem eindeutig widergespiegelt.

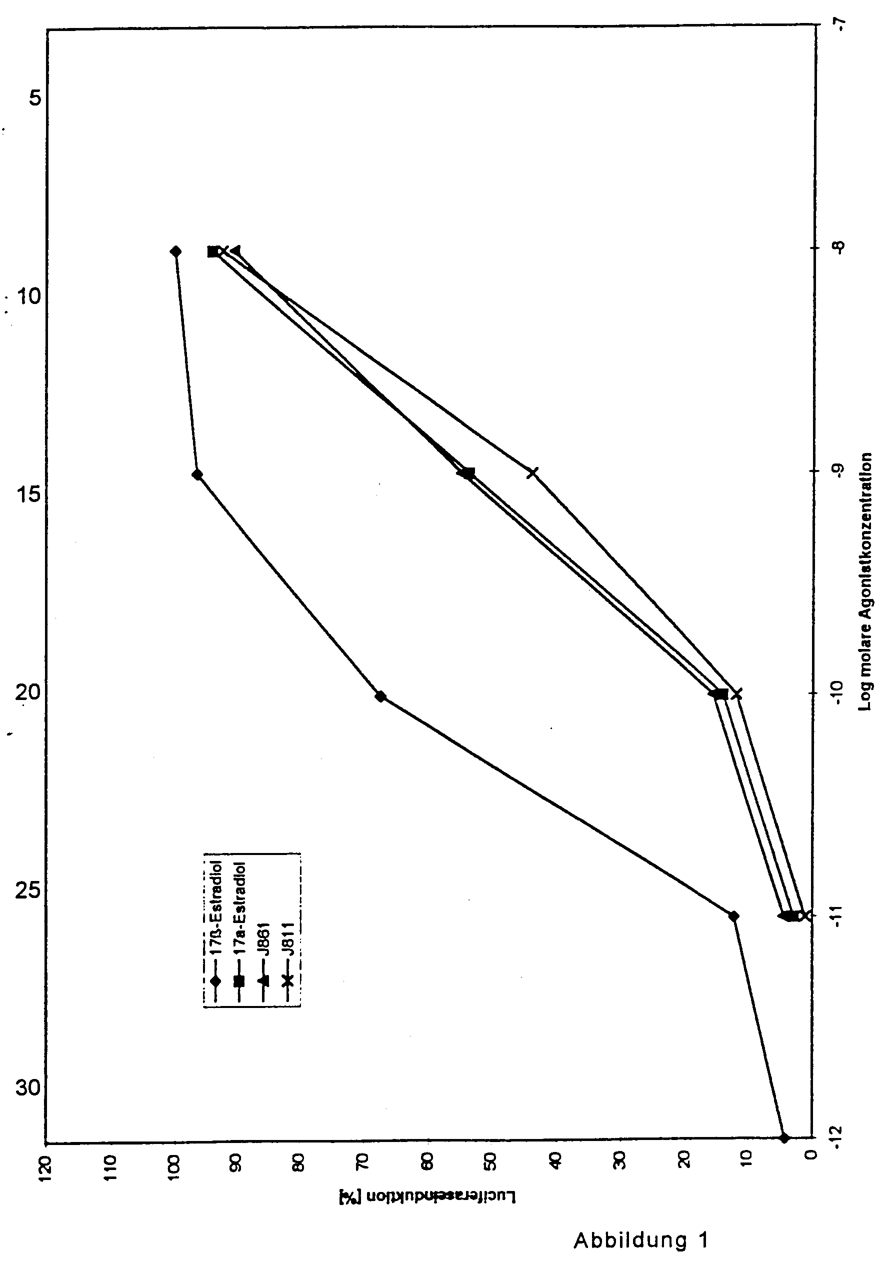

### Klinische Studie

Die aufgezeigten Systeme wurden im Rahmen einer klinischen Studie an älteren Männern erprobt.

Die Wirksamkeit von täglich 2 mg 17α-Estradiol, verabreicht in Hartgelantinekapseln, wurde an 12 übergewichtigen Männern (Körpermasse-Index ((BMI)) >35) im Alter von 52 bis 63 Jahren über 6 Monate erprobt. Keiner der Probanden nahm eine weitere Medikation ein. Bei 6 der 12 Probanden ergab sich ein mäßig veränderter oraler Glukosetoleranz-Test (OGTT), keiner zeigte jedoch eine deutliche diabetische Stoffwechsellage. Schildrüsen-, Leber- und Nierenfunktionsteste waren vor Beginn der Studie bei allen Probanden unauffällig. Auch die Plasma-Androgenwerte lagen im Normbereich Testosteron > 12 nmol/l; Freies Testosteron > 0,2 nmol/l). Wie erwartet, hatten die 6 Probanden mit verändertem OGTT erhöhte Gesamtcholesterol- und Triglycerid-Werte.

Die Blutproben für die In-vivo-Bestimmung der LDL-Cholesterol-Oxidation und die Hormonanalysen wurden zu Studienbeginn, danach wöchentlich und einen Tag nach letzter Einnahme entnommen (nach nächtlichem und morgendlichem Fasten jeweils zwischen 8 und 10 Uhr morgens).

In Tabelle 4 sind die Ergebnisse der Hormonanalysen (nmol/l) zu Versuchsbeginn und nach 6 Monaten dargestellt und mit den Werten von 12 normalgewichtigen Männern im Alter von 21 bis 28 Jahren (Kontrollgruppe) verglichen.

**Tabelle 4**

| Hormonanalysen (nmol/l) zu Versuchsbeginn und nach 6 Monaten. | | | | | | |
|---|---|---|---|---|---|---|
| Gruppe | Gesamt-Testosteron | Freies Testosteron | SHBG | DHEAS | DHEA | Insulin (mlE/L) |
| Vergleichs-Kontrolle | 21.5 ± 6.3 | 0.43 ± 0.18 | 51 ± 16 | 5510 ± 2397 | 17.2 ± 9.5 | 9.8 ± 3.1 |
| Versuchsbeginn | 16.5 ± 4.6* | 0.32 ± 0.14* | 38 ± 17* | 5412 ± 3711 | 14.3 ± 10.6 | 17 ± 10 |
| Versuchsende | 15.6 ± 5.1* | 0.33 ± 0.15* | 39 ± 18* | 5660 ± 3292 | 13.46 ± 8.13 | 15 ± 9 |

| | | | | | | |
|---|---|---|---|---|---|---|
| * p < 0.05 vs. Kontrollgruppe | | | | | | |

Es wird ersichtlich, daß die Behandlung mit 17α-Estradiol die gewählten Hormonparameter nicht signifikant beeinflußt. Der fehlende SHBG-Anstieg ist ein Beleg, daß 17α-Estradiol - im Gegensatz zu 17β-Estradiol - nicht über die klassischen Estrogenwirkungen verfügt.

In Tabelle 5 ist das Verhalten des Lipid- bzw. Lipoproteinprofils zu Versuchsbeginn und am Versuchsende dargestellt.

**Tabelle 5**

| Lipid- und Lipoproteinprofil | | |
|---|---|---|
| Lipid | Versuchsbeginn | Versuchsende |
| Gesamt-Cholesterol (mg/dl) | 225 ± 11.1 | 195 ± 5.7 * |
| LDL-Cholesterol (mg/dl) | 154 ± 10.5 | 135 ± 5.2 |
| HDL-Cholesterol (mg/dl) | 33 ± 1 .5 | 38 ± 1 .5 |
| Triglyceride (mg/dl) | 226 ± 31.1 | 132 ± 1.5 * |
| Lp(a) (mg/dl) | 6.0 ± 1.0 | 5.7 ±0.7 |

| | | |
|---|---|---|
| *p<0.05 | | |

Der Abfall des Gesamtcholesterols als ein wichtiger Marker eines atherogenen Risikos ist signifikant. Der gleichfalls signifikante Abfall der Triglyceride (weiterer Risiko-Faktor ) fügt sich ebenfalls nicht in das Bild der klassischen Estrogenwirkungen ein, bei welchem meist ein Triglycerid-Anstieg, zumindest kein Triglycerid-Abfall beobachtet wird.

Keiner der Probanden berichtete unter der Behandlung über unerwünschte Wirkungen. Insbesondere wurden Brustvermehrung (Gynäkomastie) oder Brustspannen als Ausdruck einer klassischen estrogenen Wirkung nicht beobachtet. Weiterhin wurde über eine Verbesserung der Schlafqualität und der Libido berichtet.

Die gute Hemmwirkung von 17α-Estradiol auf einen gegebenen oxidativen Prozeß bei fehlender klassischer estrogener Grundwirkung lassen 17α-konfigurierte Estrogene überraschenderweise zu geeigneten Wirkstoffen in der Verwendung zur Herstellung pharmazeutischer Präparate zur medikamentösen hormonellen Substitution beim Mann werden.

Wie bereits aus der Patentliteratur bekannt, besitzt das 17α-Estradiol die gleichen antioxidativen Eigenschaften wie das hochwirksame 17β-Estradiol und das synthetische Estrogen Ethinylestradiol. Da diese antioxidativen Wirkungen auch in zellfreien Testsystemen nachgewiesen werden konnten, scheiden die klassischen (zellkernvermittelten) Wirkungsmechanismen für diese spezielle Estrogenwirkung aus.

Beim älteren Manne wird belegt, daß 17α-Estradiol keine klassischen Estrogenwirkungen zeigt, wie dies am fehlenden Anstieg des Sexualhormonbindenden Globulins (SHBG) als einem sehr empfindlichen Marker einer estrogenen Wirkung und an der fehlenden Ausbildung einer Gynäkomastie bzw. von Brustspannen nachgewiesen wird. Besonders ist die deutliche Verzögerung unter LDL-Cholesterol-Oxidation beim älteren Manne unter Gabe von 17α-Estradiol zu erwähnen. Damit kann ein wichtiger Schritt für die Entstehung und den Unterhalt einer Atheriosklerose gebremst werden, ohne daß unerwünschte estrogene Nebenwirkungen in Kauf genommen werden müissen. Darüber hinaus wurden weitere Marker eines atheriosklerotischen Risikos wie Gesamtcholesterol und Triglyceride im Blut gesenkt.

Experimentell wird aufgezeigt, daß GABAerg-vermittelte Krampfzustände im Gehirn günstig beeinflußt werden. Über GABAerge Mechanismen werden chronisch-degenerative Zustände im Gehirn und das Nachlassen kognitiver Leistungen in Verbindung gebracht. Erfindungsgemäß beanspruchte Steroide sind in der Lage, die Expression von Adhäsionsmolekülen im Endothel zu bremsen. Die vermehrte Bildung von Adhäsionsmolekülen gehört ebenfalls zu den Initialschritten bei der Auslösung von Entzündungen und von Atherosklerose.

## Patentansprüche

1. Verwendung von
- 17α-Estradiol, dessen chemisch modifizierten Derivaten oder den Estern des 17α-Estradiol oder deren chemisch modifizierten Derivaten mit antioxidativen Eigenschaften, ausgenommen Ethinylestradiol,
zur Herstellung von pharmazeutischen Präparaten zur Prophylaxe und Therapie chronisch-degenerativer Erkrankungen im Gehirn, von Atherosklerose und Arthritis/Arthrose beim Mann .

## Claims

1. Use of
- 17α-estradiol, the chemically modified derivatives thereof or the esters of 17α-estradiol or the chemically modified derivatives thereof having antioxidant properties, with the exception of ethynylestradiol,
for the production of pharmaceutical preparations for the prevention and treatment of chronic-degenerative diseases of the brain, of atherosclerosis and arthritis/osteoarthritis in humans.

## Revendications

1. Utilisation de
- 17α-oestradiol, de leurs dérivés chimiquement modifiés ou les esters du 17α-oestradiol ou leurs dérivés chimiquement modifiés avec propriétés antioxydantes à l'exception de l'éthinyloestradiol
pour la production de préparations pharmaceutiques pour la prophylaxie et la thérapie de maladies chroniques dégénératives dans le cerveau, de l'athérosclérose et de l'arthrite/arthrose chez l'homme.
